**Europäisches Patentamt**

**European Patent Office**

**Office européen des brevets**

(11) Veröffentlichungsnummer: **0 013 863**
**A1**

# EUROPÄISCHE PATENTANMELDUNG

(21) Anmeldenummer: **79810009.5**

(51) Int. Cl.³: **A 61 F 1/00**

(22) Anmeldetag: **26.01.79**

(43) Veröffentlichungstag der Anmeldung: **06.08.80**
**Patentblatt 80/16**

(71) Anmelder: **OSTEO AG, Bielstrasse 620, CH-2545 Selzach (CH)**

(72) Erfinder: **Mittelmeier, Heinz, Prof. Dr. med., An der Schutzhütte, D-Blieskastel (DE)**
Erfinder: **Moser, Heinz, Sparetweg 440, CH-2545 Selzach (CH)**
Erfinder: **Leu, Beat, Hintere Gasse 33, CH-2500 Biel (CH)**

(74) Vertreter: **Seehof, Michel et al, c/o AMMANN PATENTANWAELTE AG BERN Schwarztorstrasse 31, CH-3001 Bern (CH)**

(84) Benannte Vertragsstaaten: **BE CH DE FR GB IT LU NL SE**

(54) **Zementfrei einsetzbare Schalenprothese, insbesondere für das Hüftgelenk.**

(57) Die zementfrei einsetzbare Schalenprothese weist eine Pfannenschale (1) und eine Kopfschale auf, die mit je einem Zentralzapfen (2) und einer Anzahl kleinerer, konzentrisch um diesen angeordneten Verankerungszapfen (3) versehen sind, wobei sämtliche Zapfen sägezahnähnliche Verankerungsmittel aufweisen. Die Pfannenschale (1) kann aus kohlefaserverstärktem Kunststoff und die Kopfschale aus Aluminium-Oxyd-Keramik hergestellt sein.

Um die Verankerung zu verstärken, sind die beiden Schalen an der dem Knochen zugewandten Seite mit Apatit bzw. apatithaltigem Bioglas beschichtet.

## Zementfrei einsetzbare Schalenprothese, insbesondere für das Hüftgelenk

Die vorliegende Erfindung bezieht sich auf eine zementfrei einsetzbare Schalenprothese, insbesondere für das Hüftgelenk, bestehend aus einer Pfannen- und einer Kopfschale.

Zur Behandlung schmerzhafter Abnützungserkrankungen (Arthrose) des Hüftgelenkes wurde erstmals in den 20er Jahren von SMITH-PETERSEN eine Interpositionsschale aus verschiedenen Materialien, später hauptsächlich aus einer Kobalt-Chrom-Legierung verwendet. Kipperscheinungen, sowie Abschliff am Hüftkopf und Hüftkopfnekrosen brachten jedoch nur teilweise befriedigende Ergebnisse. Neben der Entwicklung der pilzförmigen Hüftkopf-Endoprothesen von JUDET wurden etwa 1950 auch festsitzende Hüftkopfüberzüge aus Metall und Plexiglas versucht, die zementfrei fixiert waren. Bei glatter innerer Oberfläche und ohne Zementfixation kam es jedoch vielfach zur Lockerung, bei Verwendung von Plexiglas auch zu Materialbrüchen, abgesehen von den dabei gleichfalls auftretenden Hüftkopfnekrosen, die jedoch hauptsächlich auf Durchblutungsstörungen des Hüftkopfes beruhen. Wegen der vielen Misserfolge wurde diese Form des Gelenkoberflächenersatzes mit einer derartigen Kopfkappe vorerst wieder verlassen.

Die Entwicklung verlief dann zunächst in Richtung auf einen ausgedehnteren Gelenkersatz unter Entfernung von Hüttkopf und Schenkelhals, mit sogenannten Teilprothesen, vorwiegend aus Metall, die mit einem intramedullären Verankerungsstiel befestigt wurden (MOORE, THOMSON). Dabei traten jedoch teilweise Abschliff- und Resorptionserscheinungen an der natürlichen Pfanne auf, was zur "Total-Endoprothese" mit zusätzlichem Oberflächenersatz der Hüftgelenkspfanne führte. Anfangs wurde eine dünne Schale aus Metall verwendet (McKEE-FARRAR), danach Kunststoff, vorwiegend Polyäthylen (CHARNLEY u.a., etwa seit 1960). Insbesondere kam dann noch zur besseren Befestigung die Verankerung mit Knochenzement (vorwiegend Polymethylmethacrylat) hinzu (CHARNLEY).

Die Erfahrung hat jedoch gezeigt, dass es infolge von Polyäthylenabrieb und vor allem Zementzerbröckelung, aber auch Fremdkörperreaktionen des Knochenzementes in einem grösseren Prozentsatz zu aseptischen Prothesenlockerungen kommt, welche oftmals zur bleibenden Prothesenentfernung zwingen, insbesondere wenn das Knochengewebe infolge der Lockerungseffekte und chemischen Zementwirkung stark zerstört ist. Es bleibt dann der funktionell nicht recht befriedigende Zustand einer sogenannten Resektionshüfte zurück, der mit Beinverkürzung und einer Instabilität im Hüftgelenk einhergeht.

Im Hinblick darauf und in der Erkenntnis der Tatsache, dass der Oberflächenersatz des Hüftgelenkes mit den Kappen-Prothesen vor allem auf die ungenügende Befestigung der Kappe zurückzuführen ist, besteht seit einigen Jahren die Tendenz zum Oberflächenersatz des Hüftgelenkes zurückzukehren. Sowohl bei der von FREEMAN seit 1972 entwickelten "Doppel-Cup"-Prothese als auch der von WAGNER 1974 eingeführten "Schalen-Prothese" wird in praktisch gleichartiger Weise der Hüftkopf an der Oberfläche halbkugelförmig zugefräst (ansonsten jedoch erhalten) und mittels Zementfixation eine halbkugelige Metallkappe aufgesetzt. An der Hüftpfanne wird
nach halbkugeliger Ausfräsung gleichfalls mittels Knochenzement eine korrespondierende Pfannenschale aus Polyäthylen
eingesetzt. Im Falle eines Misserfolges durch Prothesenlockerung besteht dann die Möglichkeit, in zweiter Sitzung,
nach Entfernung der Prothesenteile, noch eine Versteifungsoperation ohne wesentliche Beinverkürzung oder einen Hüftgelenksersatz mit einer herkömmlichen Totalprothese, unter
zusätzlicher Entfernung von Hüftkopf und Schenkelhals
vorzunehmen. Vor allem in dieser Rückzugsmöglichkeit wird
ein Vorteil des Oberflächenersatzes im Vergleich zu den herkömmlichen Typen des Hüftgelenkersatzes gesehen.

Gegenüber dem herkömmlichen Hüftgelenkersatz hat die Doppel-
Schalen-Prothese dieser Art jedoch einige konstruktive
Nachteile, die Befürchtungen hinsichtlich der Haltbarkeit
ergeben. Dies gilt zunächst bezüglich des Pfannenverschleisses, da der im Vergleich zu den herkömmlichen Total-Prothesen grosse Radius auch ein grosses Reibungsdrehmoment
und eine relativ hohe Oberflächengeschwindigkeit beinhaltet.

Im Vergleich zu herkömmlichen Prothesen mit relativ kleinem Kopfdurchmesser, besteht bei dünnen Pfannenschalen somit die Gefahr, in nicht allzu langer Zeit durchgerieben zu
werden, zumal bei der Paarung Metall/Polyäthylen vergleichsweise hohe Verschleissraten bestehen. Zur Herabsetzung der
Reibung wird deshalb neuerdings bei der Wagner-Schalen-
Prothese die Hüftkopfkappe aus Aluminium-Oxyd-Keramik hergestellt, was nach bisherigen Erkenntnissen über die Tribologie der Endoprothesen eine Verschleissminderung beinhaltet. Andererseits besteht bei dünnen Keramik-Schalen
jedoch eine erhöhte Bruchgefahr.

Zum anderen besteht beim Oberflächenersatz mit Zementfixation
das bekannte Langzeitrisiko des Knochenzementes fort, dass eventuell infolge der toxischen Wirkung durch Monomerabgabe langfristig eine maligne Gewebsentartung entstehen könnte. Da nach vorherrschender Meinung der Knochenzement deshalb möglichst nur in höherem Alter Verwendung finden sollte, können die bislang gebräuchlichen zementierbaren Schalenprothesen bei jüngeren Menschen nur mit Vorbehalt Anwendung finden. Vor allem aber bestehen Bedenken, dass die nur relativ dünne Zementschicht zwischen den Schalen und dem Knochengewebe bei der bekannt niedrigen Dauerschwingfestigkeit des herkömmlichen Knochenzementes zur aseptischen Prothesenlockerung führt. Dazu dürfte u. E. insbesondere der niedrige Elastizitätsmodul des für die Pfanne verwendeten Polyäthylens beitragen, das bei Belastung eine erhebliche Verformung erfährt, welches sich auch auf die Zementschicht mit relativ starken Verformungen auswirkt.

Es ist demgegenüber das Ziel der vorliegenden Erfindung eine zementfrei einsetzbare Schalenprothese anzugeben, die fest im Knochen verankert werden kann und die gute Gleiteigenschaften aufweist und einer sehr geringen Abnützung unterworfen ist.

Bei einer solchen Schalenprothese weisen sowohl die Pfannenals auch die Kopfschale je einen Zentralzapfen und eine Anzahl kleinere, konzentrisch darum angeordnete Verankerungszapfen auf, wobei sämtliche Zapfen Verankerungsmittel in Form von sägezahnähnlichen Kerben oder eines Knochenschraubenprofils aufweisen. In bevorzugten Ausführungsbeispielen ist die Pfannenschale aus mit kohlefaserverstärkten Kunststoff hergestellt und die Kopfschale entweder aus körperverträglichem Metall oder vorzugsweise aus Aluminium-Keramik hergestellt. Um das Verhaften der Schalen zu verbessern, sind die dem Knochen zugewandten Seiten der beiden Schalen mit das Anwachsen des Knochens fördernden Materialien wie Apatit oder Glaskeramik beschichtet.

Die Erfindung wird im folgenden anhand einer Zeichnung eines Ausführungsbeispiels näher erläutert werden. Es zeigen

Figur 1 eine Pfannenschale im Schnitt,

Figur 2, in Draufsicht, die dem Knochen zugewandte Seite der Pfannenschale von Figur 1,

Figur 3, im verkleinerten Massstab, eine Kopfschale im Schnitt und

Figur 4, im vergrösserten Massstab und im Schnitt, einen im Knochen eingesetzten Zapfen.

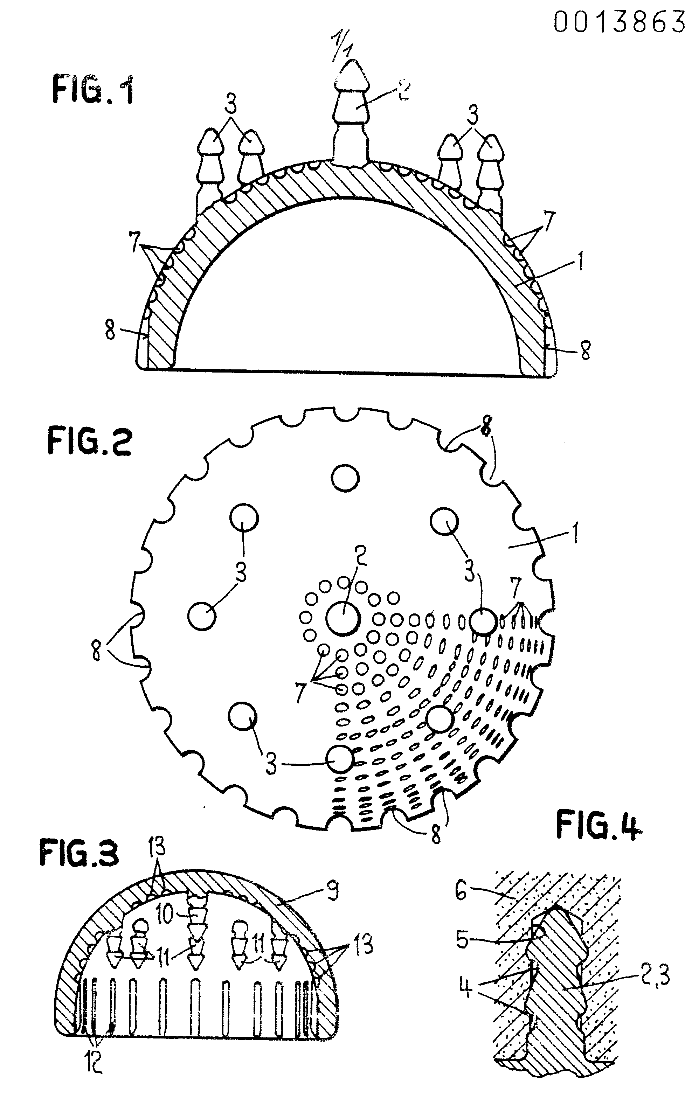

In den Figuren 1 und 2 erkennt man eine Pfannenschale 1 mit dem Zentralzapfen 2 und die konzentrisch um diesen angeordneten Verankerungszapfen 3, die etwas kleiner sind als der Zentralzapfen. Wie auch aus Figur 4 hervorgeht, weisen die Zapfen 2 und 3 ein sägezahnähnliches Profil mit Einkerbungen 4 auf, wodurch, falls die Bohrung 5 (Figur 4) im Knochen kleiner ist als der äussere Durchmesser der Zapfen, ein Effekt wie bei Widerhaken erzielt wird und eine gute Verankerung ermöglicht wird. Falls der Durchmesser der Einkerbung 4 kleiner als die Bohrung im Knochen 6 gewählt wird, kann dieser dann in diese Einkerbung hineinwachsen. Der längere und dickere Zapfen 2 dient insbesondere auch der Zentrierung der Pfannenschale beim Einsetzen. Die äussere, knochenseitige Oberfläche der Schale weist zwecks weiterer Verzahnung und Oberflächenvergrösserung eine wabenförmige Struktur auf, wobei in vorliegendem Fall die Höhlungen 7 etwa Stecknadelkopfgrösse erreichen. Zur Verbesserung der Drehstabilität sind am Umfang der Schale parallel zur Schalenachse verlaufende Nuten 8 angebracht. Anstatt Nuten können jedoch ebensogut Rippen angeordnet sein. Die Kopfschale 9 ist analog der Pfannenschale 1 aufgebaut und weist ebenfalls einen parallel zur Schalenachse verlaufenden
Zentralzapfen 10 sowie konzentrisch dazu angeordnete Verankerungszapfen 11 auf, die die gleiche Struktur wie in
Figur 4 erläutert aufweisen. Am inneren Umfang dieser Schale
sind parallel zur Schalenachse verlaufende Nuten 12 oder
Rippen angebracht, die der Erhöhung der Drehstabilität
dienen. Die Innenseite ist ebenfalls mit Höhlungen 13 versehen.

Die Pfannenschale 1 wird aus kohlefaserverstärkten Polyäthylen hergestellt, wobei der Anteil, die Verteilung und
gegebenenfalls die Anordnungen der Kohlefasern im Kunststoff nicht für alle Teile gleich sein muss und insbesondere die zentralen Teile der Zapfen zweckmässigerweise
einen höheren Anteil an Kohlenstoff-Fasern aufweisen können.
Die Kopfschale 9 kann auch aus kohlefaserverstärkten Kunststoff bestehen oder aber auch aus einem körperverträglichen
Metall, wie einer Chrom-Kobalt-Legierung oder aus Aluminium-
Oxyd-Keramik. Die Verwendung von Aluminiumoxyd-Keramik erlaubt es insbesondere, die Gelenkoberfläche genau und mit
einer sehr geringen Rauhigkeit zu bearbeiten, wodurch ein
sehr gutes Reibungsverhalten und ein sehr geringer Abrieb
erzielt wird.

Eine noch bessere Verankerung der Prothesenschalen ergibt
sich dadurch, dass die dem Knochen zugewandten Oberflächen
mit bioaktiven, nicht resorbierbaren, apatithaltigen
Materialien beschichtet werden, welche ein unmittelbares Anwachsen des Knochengewebes ohne bindegewebige Grenzmembran
und damit eine zusätzlich chemisch physikalische Bindung des
Implantates an das Knochengewebe ermöglichen. Dazu kommen
insbesondere Partikel aus apatithaltigem Bioglas, Apatit
oder enteiweisstem hetero-, homöo- oder autoplastischem
Knochengewebe in Frage. Im Falle eines Prothesenteils aus
faserverstärktem Kunststoff können diese Partikel während
dem Herstellungsprozess oder in einem weiteren Arbeitsgang in die in Frage kommende Oberfläche eingebracht werden,
wobei dafür Sorge getragen werden muss, dass die Partikel
überäquatorial eingefasst werden, um nicht herauszufallen.
Bei der Verwendung einer Kopfschale aus Aluminium-Oxyd-
Keramik ist eine Beschichtung aus bioaktivem Glas am geeignetsten, wobei diese Schicht nicht zusammenhängend sein
soll, um deren Zerbröckeln zu verhindern.

Um das Einsetzen der Schalen am Knochen zu erleichtern,
ist es zweckmässig, mittels einer speziellen Schablone
an den den Verankerungszapfen entsprechenden Stellen die
Bohrungen anzubringen, wobei deren Durchmesser, wie aus
Figur 4 insbesondere hervorgeht, zwischen dem Durchmesser
der Ausbuchtungen und dem Durchmesser der Einschnürung
liegen muss. Zweckmässigerweise werden die Bohrer mit einer
Einrichtung zur Begrenzung der Tiefe der Bohrlöcher versehen.

Um einer Anpassung der verschiedenen Grössen der natürlichen
Hüftgelenke zu gewährleisten, müssen die Schalen in entsprechenden Grössen vorliegen.

- 1 -

Patentansprüche:

1. Zementfrei einsetzbare Schalenprothese, insbesondere
   für das Hüftgelenk, bestehend aus einer Pfannen- und
   einer Kopfschale,
   gekennzeichnet durch
   je einen Zentralzapfen (2, 10) und einer Anzahl kleinerer,
   konzentrisch darum angeordneter Verankerungszapfen (3,
   11), wobei sämtliche Zapfen Verankerungsmittel in Form
   von sägezahnähnlichen Kerben (6) oder eines Knochenschraubenprofils aufweisen.

2. Schalenprothese nach Anspruch 1,
   dadurch gekennzeichnet,
   dass die beiden Schalen (1, 9) an der dem Knochen (6)
   zugewandten Seite eine wabenförmige Struktur (7) aufweisen.

3. Schalenprothese nach Anspruch 1 oder 2,
   dadurch gekennzeichnet,
   dass die beiden Schalen (1, 9) an ihrem Umfang parallel
   zur Schalenachse verlaufende Nuten (8, 12) oder Rippen
   aufweisen.

4. Schalenprothese nach einem der Ansprüche 1 - 3,
   dadurch gekennzeichnet,
   dass mindestens die Pfannenschale (1) aus mit Kohle-

faserr verstärktem Kunststoff herg stellt ist.

5. Schalenprothese nach einem der Ansprüche 1 - 4,
   dadurch gekennzeichnet,
   dass die Kopfschale (9) aus körperverträglichem Metall
   oder Aluminiumkeramik hergestellt ist.

6. Schalenprothese nach einem der Ansprüche 1 - 5,
   dadurch gekennzeichnet,
   dass die beiden Schalen an der dem Knochen zugewandten
   Seite mit die Verhaftung des Knochens fördernden nicht
   resorbierbaren Materialien beschichtet sind.

7. Schalenprothese nach einem der Ansprüche 1 - 6,
   dadurch gekennzeichnet,
   dass die Schalen aus kohlefaserverstärktem Kunststoff
   mit Apatit oder enteiweisstem Knochengewebe beschichtet
   sind, wobei die Partikel überäquatorial in der Oberfläche
   verankert sind und keine zusammenhängende Schicht bilden.

8. Schalenprothese nach einem der Ansprüche 1 - 7,
   dadurch gekennzeichnet,
   dass die Schalen aus Aluminiumoxydkeramik mit bioaktivem
   Glas in Partikelform beschichtet sind, wobei die Partikel
   keine zusammenhängende Schicht bilden.

FIG.1

FIG.2

FIG.3

FIG.4

0013863

| Kategorie | EINSCHLÄGIGE DOKUMENTE | | KLASSIFIKATION DER ANMELDUNG (Int.Cl.²) |
|---|---|---|---|
| | Kennzeichnung des Dokuments mit Angabe, soweit erforderlich der maßgeblichen Teile | betrifft Anspruch | |
| X | <u>FR - A - 2 301 217</u> (CERAVER)<br>* Seite 2, Zeilen 12 bis 21;<br>  Ansprüche 1, 2; Fig. 1, 2<br>  Positionen 5, 6, 7 *<br><br>-- | 1-3 | A 61 F   1/00 |
| | <u>DE - A - 2 259 313</u> (CERAVER)<br>* Seite 3, Absatz 2; Seite 4,<br>  Absätze 4, 5; Anspruch 3; Fig. 1 *<br><br>-- | 1 | |
| | <u>DE - C - 976 768</u> (F.D. TIMMERMANS)<br>* Ansprüche 5, 9, 13; Fig. 1,<br>  Positionen c, p, p' *<br><br>-- | 1 | **RECHERCHIERTE SACHGEBIETE (Int. Cl.²)**<br><br>A 61 F   1/0C |
| | <u>DE - A - 2 501 683</u> (E. LEITZ)<br>* Seite 3, Zeilen 30 bis 33;<br>  Seite 5, Zeilen 12 bis 13 und<br>  24 bis 34; Anspruch 2 *<br><br>-- | 4,6,7 | |
| | <u>DE - A - 2 708 917</u> (R. BOSCH)<br>* Anspruch 1 *<br><br>-- | 4,6 | **KATEGORIE DER GENANNTEN DOKUMENTE** |
| | <u>DE - A - 2 535 649</u> (ROSENTHAL TECHNIK)<br>* Ansprüche 4, 7 *<br><br>--<br>./.. | 5 | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 16-08-1979 | DROPMANN |

EPA form 1503.1  06.78

Europäisches
Patentamt

EUROPÄISCHER RECHERCHENBERICHT

0013863
Nummer der Anmeldung

EP 79 810 009.5
- Seite 2 -

## EINSCHLÄGIGE DOKUMENTE

| | KLASSIFIKATION DER ANMELDUNG (Int.Cl.²) |
|---|---|

| Kategorie | Kennzeichnung des Dokuments mit Angabe, soweit erforderlich, der maßgeblichen Teile | betrifft Anspruch |
|---|---|---|
| | DE - A - 2 625 529 (SIGRI ELEKTRO-GRAPHIT) <br> * Anspruch 1 * <br><br> -- | 6,7 |
| | DE - A - 2 313 678 (H. BECKER et al.) <br> * Ansprüche 1, 2; Seite 3, Absatz 1 * <br><br> -- | 6 |
| | DE - A - 2 546 824 (E. LEITZ) <br> * Seite 9, Absätze 2 und 4; <br> Fig. 1 * <br><br> -- | 8 |
| A | FR - A - 2 391 712 (SULZER FRERES) <br> * Fig. 1 * <br><br> -- | |
| A | DE - A - 2 559 402 (PFAUDLER-WERKE) <br> * Anspruch 1 * <br><br> ---- | |

RECHERCHIERTE
SACHGEBIETE (Int. Cl.²)